# EUROPEAN PATENT SPECIFICATION

(11) **EP 2 222 346 B1**
(45) Date of publication and mention of the grant of the patent: **08.02.2012**
(21) Application number: 08864838.1
(22) Date of filing: 19.12.2008
(51) Int. Cl.: A61K 49/10, A61K 49/20

(54) **MR IMAGING AGENT, IMAGING MEDIUM AND METHODS OF IMAGING WHEREIN SUCH AN IMAGING MEDIUM IS USED**
MAGNETRESONANZ-ABBILDUNGSMITTEL, ABBILDUNGSMEDIUM UND VERFAHREN ZUR ABBILDUNG, WORIN SOLCH EIN ABBILDUNGSMEDIUM VERWENDET WIRD
AGENT D'IMAGERIE PAR RÉSONANCE MAGNÉTIQUE, SUPPORT D'IMAGERIE ET PROCÉDÉS D'IMAGERIE DANS LESQUELS UN TEL SUPPORT D'IMAGERIE EST UTILISÉ

(30) Priority: 21.12.2007 NO 20076640
(43) Date of publication of application: 01.09.2010
(73) Proprietor: GE Healthcare Limited, Little Chalfont Buckinghamshire HP7 9NA (GB)
(72) Inventor: GISSELSSON, Anna, S-SE-227 63 Lund (SE); HANSSON, Georg, S-SE-235 35 Vellinge (SE); MÅNSSON, Sven, S-SE-237 34 Bjärred (SE); IN'T ZANDT, René, S-SE-247 36 Södra Sandby (SE); KARLSSON, Magnus, S-SE-212 43 Malmö (SE); JENSEN, Pernille, R., DK-2200 København N (DK); LERCHE, Mathilde, H., DK-1903 Fredriksberg C (DK)
(74) Representative: Wulff, Marianne Weiby
(86) International application number: PCT/EP2008/067986
(87) International publication number: WO 2009/080739

(56) References cited:
- WO-A-2006/011810
- WO-A-2007/064226
- WO-A-2007/069909
- WO-A-2008/086534
- HEISSIG HENRIKE ET AL: "Mechanism of the insulin-releasing action of alpha-ketoisocaproate and related alpha-keto acid anions" MOLECULAR PHARMACOLOGY, vol. 68, no. 4, October 2005 (2005-10), pages 1097-1105 URL, XP002522443 ISSN: 0026-895X

## Description

The invention relates to hyperpolarised ¹³C-α-ketoisocaproate, its use as imaging agent, an imaging medium comprising hyperpolarised ¹³C-α-ketoisocaproate and such an imaging medium for use in methods of ¹³C-MR detection. Further, the invention relates to methods of producing hyperpolarised ¹³C-α-ketoisocaproate.

Magnetic resonance (MR) imaging (MRI) is a technique that has become particularly attractive to physicians as images of a patients body or parts thereof can be obtained in a non-invasive way and without exposing the patient and the medical personnel to potentially harmful radiation such as X-rays. Because of its high quality images and good spatial and temporal resolution, MRI is a favourable imaging technique for imaging soft tissue and organs.

MRI may be carried out with or without MR contrast agents. However, contrast-enhanced MRI usually enables the detection of much smaller tissue changes which makes it a powerful tool for the detection of early stage tissue changes like for instance small tumours or metastases.

Several types of contrast agents have been used in MRI. Water-soluble paramagnetic metal chelates, for instance gadolinium chelates like Omniscan^{™} (GE Healthcare) are widely used MR contrast agents. Because of their low molecular weight they rapidly distribute into the extracellular space (i.e. the blood and the interstitium) when administered into the vasculature. They are also cleared relatively rapidly from the body.

Blood pool MR contrast agents on the other hand, for instance superparamagnetic iron oxide particles, are retained within the vasculature for a prolonged time. They have proven to be extremely useful to enhance contrast in the liver but also to detect capillary permeability abnormalities, e.g. "leaky" capillary walls in tumours which are a result of tumour angiogenesis.

Despite the undisputed excellent properties of the aforementioned contrast agents their use is not without any risks. Although paramagnetic metal chelates have usually high stability constants, it is possible that toxic metal ions are released in the body after administration. Further, these type of contrast agents show poor specificity.

WO-A-99/35508 discloses a method of MR investigation of a patient using a hyperpolarised solution of a high T₁ agent as MRI contrast agent. The term "hyperpolarisation" means enhancing the nuclear polarisation of NMR active nuclei present in the high T₁ agent, i.e. nuclei with non-zero nuclear spin, preferably ¹³C- or ¹⁵N-nuclei. Upon enhancing the nuclear polarisation of NMR active nuclei, the population difference between excited and ground nuclear spin states of these nuclei is significantly increased and thereby the MR signal intensity is amplified by a factor of hundred and more. When using a hyperpolarised ¹³C- and/or ¹⁵N-enriched high T₁ agent, there will be essentially no interference from background signals as the natural abundance of ¹³C and/or ¹⁵N is negligible and thus the image contrast will be advantageously high. The main difference between conventional MRI contrast agents and these hyperpolarised high T₁ agents is that in the former changes in contrast are caused by affecting the relaxation times of water protons in the body whereas the latter class of agents can be regarded as non-radioactive tracers, as the signal obtained arises solely from the agent.

A variety of possible high T₁ agents for use as MR imaging agents are disclosed in WO-A-99/35508, including non-endogenous and endogenous compounds. As examples of the latter intermediates in normal metabolic cycles are mentioned which are said to be preferred for imaging metabolic activity. By *in vivo* imaging of metabolic activity, information of the metabolic status of a tissue may be obtained and said information may for instance be used to discriminate between healthy and diseased tissue.

For instance pyruvate is a compound that plays a role in the citric acid cycle and the conversion of hyperpolarised ¹³C-pyruvate to its metabolites hyperpolarised ¹³3C-lactate, hyperpolarised ¹³C-bicarbonate and hyperpolarised ¹³C-alanine can be used for *in vivo* MR studying of metabolic processes in the human body.

The metabolic conversion of hyperpolarised ¹³C-pyruvate to its metabolites hyperpolarised ¹³C-lactate, hyperpolarised ¹³C-bicarbonate and hyperpolarised ¹³C-alanine can be used for *in vivo* MR study of metabolic processes in the human body since said conversion has been found to be fast enough to allow signal detection from the parent compound, i.e. hyperpolarised ¹³C₁-pyruvate, and its metabolites. The amount of alanine, bicarbonate and lactate is dependent on the metabolic status of the tissue under investigation. The MR signal intensity of hyperpolarised ¹³C-lactate, hyperpolarised ¹³C-bicarbonate and hyperpolarised ¹³C-alanine is related to the amount of these compounds and the degree of polarisation left at the time of detection, hence by monitoring the conversion of hyperpolarised ¹³C-pyruvate to hyperpolarised ¹³C-lactate, hyperpolarised ¹³C-bicarbonate and hyperpolarised ¹³C-alanine it is possible to study metabolic processes *in vivo* in the human or non-human animal body by using non-invasive MR imaging and/or MR spectroscopy.

The MR signal amplitudes arising from the different pyruvate metabolites vary depending on the tissue type. The unique metabolic peak pattern formed by alanine, lactate, bicarbonate and pyruvate can be used as fingerprint for the metabolic state of the tissue under examination.

Hyperpolarised ¹³C-pyruvate may for instance be used as an MR imaging agent for assessing the viability of myocardial tissue by MR imaging as described in detail in WO-A-2006/054903 and for *in vivo* tumour imaging as described in detail in WO-A-2006/011810.

Tumour tissue is often characterised by an increased perfusion and higher metabolic activity. The process of increasing the vascular bed, angiogenesis, is induced by cells that due to their higher metabolic needs and/or their larger distance from a capillary are not able to get enough substrates that can provide the energy needed to sustain energy homeostasis. It is in this area, where cells have problems in producing enough energy a marked change in metabolic pattern is expected. Tissue with problems sustaining energy homeostasis will alter its energy metabolism which in particular results in an increased lactate production. With the use of hyperpolarised ¹³C-pyruvate as an MR imaging agent, this higher metabolic activity can be seen by an increased production of ¹³C-lactate which can be detected by ¹³C-MR detection.

However, since the production of hyperpolarised ¹³C-pyruvate which is suitable as an *in vivo* imaging agent is not without challenges, there is a need of alternative hyperpolarised imaging agents which can be used to obtain information about metabolic activity, especially in the field of oncology.

We have now found that hyperpolarised ¹³C-α-ketoisocaproate may be used as such an imaging agent.

α-Ketoisocaproic acid is reversibly metabolized to leucine; the enzyme branched chain aminotransferase catalyses said reaction and glutamate/α-ketoglutarate is needed as co-substrates. Further, decarboxylation of α-ketoisocaproic acid by branched chain α-ketoacid dehydrogenase results in the formation of CO₂ and subsequently bicarbonate. Both of these metabolic conversions of α-ketoisocaproic acid take place in the mitochondrion. Hence by using hyperpolarised ¹³C-α-ketoisocaproate as an imaging agent, the metabolic activity can be assessed.

Thus in a first aspect the invention provides an imaging medium comprising hyperpolarised ¹³C-α-ketoisocaproate

The term "imaging medium" denotes a liquid composition comprising hyperpolarised ¹³C-α-ketoisocaproate as the MR active agent, i.e. imaging agent.

The imaging medium according to the claims may be used as an imaging medium for *in vivo* ¹³C-MR detection, i.e. in living human or non-human animal beings. Further, the imaging medium according to the claims may be used as imaging medium for *in vitro* ¹³C-MR detection, e.g. of cell cultures, samples like for instance urine, saliva or blood, *ex vivo* tissue, for instance ex *vivo* tissue obtained from a biopsy or isolated organs, all of those derived from a living human or non-human animal body. In a preferred embodiment, the imaging medium according to the claims may be used as an imaging medium for *in vivo* ¹³C-MR detection

The term "¹³C-MR detection" denotes ¹³C-MR imaging or ¹³C-MR spectroscopy or combined ¹³C-MR imaging and ¹³C-MR spectroscopy, i.e. ¹³C-MR spectroscopic imaging. The term further denotes ¹³C-MR spectroscopic imaging at various time points.

The term "¹³C-α-ketoisocaproate" denotes a salt of 4-methyl-2-oxopentanoic acid, i.e. a salt comprising 4-methyl-2-oxopentanoate as an anion and said salt is isotopically enriched with ¹³C.

The isotopic enrichment of the hyperpolarised ¹³C-α-ketoisocaproate is preferably at least 75%, more preferably at least 80% and especially preferably at least 90%, an isotopic enrichment of over 90% being most preferred. Ideally, the enrichment is 100%. Generally, hyperpolarised ¹³C-α-ketoisocaproate according to the invention may be isotopically enriched at any carbon atom in the molecule. However, to achieve a long T1, it is preferred that ¹³C-α-ketoisocaproate is isotopically enriched with ¹³C at the C1-position (in the following denoted ¹³C₁-α-ketoisocaproate) or at the C2-position (in the following denoted ¹³C₂-α-ketoisocaproate) or in the C4-position (in the following denoted ¹³C₄-α-ketoisocaproate). Multiple enrichment is also possible like isotopic enrichment at both the C1- and C2-position (in the following denoted ¹³C_{1,2}-α-ketoisocaproate), at the C1- and the C4-position (in the following denoted ¹³C_{1,4}-α-ketoisocaproate) or at the C1-, C2- and C4-position (in the following denoted ¹³C_{1,2,4}-α-ketoisocaproate). Isotopic enrichment at the C1-position is preferred.

The terms "hyperpolarised" and "polarised" are used interchangeably hereinafter and denote a nuclear polarisation level in excess of 0.1%. more preferred in excess of 1% and most preferred in excess of 10%.

The level of polarisation may for instance be determined by solid state ¹³C-NMR measurements in solid hyperpolarised ¹³C-α-ketoisocaproate, e.g. solid hyperpolarised ¹³C-α-ketoisocaproate obtained by dynamic nuclear polarisation (DNP) of ¹³C-α-ketoisocaproate. The solid state ¹³C-NMR measurement preferably consists of a simple pulse-acquire NMR sequence using a low flip angle. The signal intensity of the hyperpolarised ¹³C-α-ketoisocaproate in the NMR spectrum is compared with signal intensity of ¹³C-α-ketoisocaproate in a NMR spectrum acquired before the polarisation process. The level of polarisation is then calculated from the ratio of the signal intensities of before and after polarisation.

In a similar way, the level of polarisation for dissolved hyperpolarised ¹³C-α-ketoisocaproate may be determined by liquid state NMR measurements. Again the signal intensity of the dissolved hyperpolarised ¹³C-α-ketoisocaproate is compared with the signal intensity of the dissolved ¹³C-α-ketoisocaproate before polarisation. The level of polarisation is then calculated from the ratio of the signal intensities of ¹³C-α-ketoisocaproate before and after polarisation.

Hyperpolarisation of NMR active ¹³C-nuclei may be achieved by different methods which are for instance described in described in WO-A-98/30918, WO-A-99/24080 and WO-A-99/35508 and hyperpolarisation methods known in the art are polarisation transfer from a noble gas, "brute force", spin refrigeration, the parahydrogen method and dynamic nuclear polarisation (DNP).

Hyperpolarised ¹³C-α-ketoisocaproate can be obtained by directly polarising ¹³C-α-ketoisocaproate or by polarisation of ¹³C-α-ketoisocaproic acid and subsequent conversion (neutralisation) of the acid to ¹³C-α-ketoisocaproate with a base. Since neutralisation with a base is an additional step, it is preferred to directly polarise ¹³C-α-ketoisocaproate. Suitable ¹³C-α-ketoisocaproates are commercially available or can be prepared from commercially available ¹³C-α-ketoisocaproic acid/¹³C-α-ketoisocaproates and will be discussed in detail in the following paragraphs.

One way for obtaining hyperpolarised ¹³C-α-ketoisocaproic acid/¹³C-α-ketoisocaproate is the polarisation transfer from a hyperpolarised noble gas which is described in WO-A-98/30918. Noble gases having non-zero nuclear spin can be hyperpolarised by the use of circularly polarised light. A hyperpolarised noble gas, preferably He or Xe, or a mixture of such gases, may be used to effect hyperpolarisation of ¹³C-nuclei. The hyperpolarised gas may be in the gas phase, it may be dissolved in a liquid/solvent, or the hyperpolarised gas itself may serve as a solvent. Alternatively, the gas may be condensed onto a cooled solid surface and used in this form, or allowed to sublime. Intimate mixing of the hyperpolarised gas with ¹³C-α-ketoisocaproic acid/¹³C-α-ketoisocaproate is preferred.

Another way for obtaining hyperpolarised ¹³C-α-ketoisocaproic acid/¹³C-α-ketoisocaproate is that polarisation is imparted to ¹³C-nuclei by thermodynamic equilibration at a very low temperature and high field. Hyperpolarisation compared to the operating field and temperature of the NMR spectrometer is effected by use of a very high field and very low temperature (brute force). The magnetic field strength used should be as high as possible, suitably higher than 1 T, preferably higher than 5 T, more preferably 15 T or more and especially preferably 20 T or more. The temperature should be very low, e.g. 4.2 K or less, preferably 1.5 K or less, more preferably 1.0 K or less, especially preferably 100 mK or less.

Another way for obtaining hyperpolarised ¹³C-α-ketoisocaproic acid/¹³C-α-ketoisocaproate is the spin refrigeration method. This method covers spin polarisation of a solid compound or system by spin refrigeration polarisation. The system is doped with or intimately mixed with suitable crystalline paramagnetic materials such as Ni²⁺, lanthanide or actinide ions with a symmetry axis of order three or more. The instrumentation is simpler than required for DNP with no need for a uniform magnetic field since no resonance excitation field is applied. The process is carried out by physically rotating the sample around an axis perpendicular to the direction of the magnetic field. The pre-requisite for this method is that the paramagnetic species has a highly anisotropic g-factor. As a result of the sample rotation, the electron paramagnetic resonance will be brought in contact with the nuclear spins, leading to a decrease in the nuclear spin temperature. Sample rotation is carried out until the nuclear spin polarisation has reached a new equilibrium.

In a preferred embodiment, DNP (dynamic nuclear polarisation) is used to obtain hyperpolarised ¹³C-α-ketoisocaproic acid/¹³C-α-ketoisocaproate. In DNP, polarisation of MR active nuclei in a compound to be polarised is affected by a polarisation agent or so-called DNP agent, a compound comprising unpaired electrons. During the DNP process, energy, normally in the form of microwave radiation, is provided, which will initially excite the DNP agent. Upon decay to the ground state, there is a transfer of polarisation from the unpaired electron of the DNP agent to the NMR active nuclei of the compound to be polarised, e.g. to the ¹³C nuclei in ¹³C-α-ketoisocaproic acid/¹³C-α-ketoisocaproate. Generally, a moderate or high magnetic field and a very low temperature are used in the DNP process, e.g. by carrying out the DNP process in liquid helium and a magnetic field of about I T or above. Alternatively, a moderate magnetic field and any temperature at which sufficient polarisation enhancement is achieved may be employed. The DNP technique is for example further described in WO-A-98/58272 and in WO-A-01/96895.

To polarise a chemical entity, i.e. compound, by the DNP method, a composition of the compound to be polarised and a DNP agent is prepared which is then optionally frozen and inserted into a DNP polariser (where it will freeze if it has not been frozen before) for polarisation. After the polarisation, the frozen solid hyperpolarised composition is rapidly transferred into the liquid state either by melting it or by dissolving it in a suitable dissolution medium. Dissolution is preferred and the dissolution process of a frozen hyperpolarised composition and suitable devices therefore are described in detail in WO-A-02/37132. The melting process and suitable devices for the melting are for instance described in WO-A-02/36005.

In order to obtain a high polarisation level in the compound to be polarised said compound and the DNP agent need to be in intimate contact during the DNP process. This is not the case if the composition crystallizes upon being frozen or cooled. To avoid crystallization, either glass formers need to be present in the composition or compounds need to be chosen for polarisation which do not crystallize upon being frozen but rather form a glass.

In one embodiment, ¹³C-α-ketoisocaproic acid, preferably ¹³C₁-α-ketoisocaproic acid is used as a starting material to obtain hyperpolarised ¹³C-α-ketoisocaproic acid by the DNP method which is then neutralised and converted to hyperpolarised ¹³C-α-ketoisocaproate with the help of a base. In another embodiment, ¹³C-α-ketoisocaproate, preferably ¹³C₁-α-ketoisocaproate is used as a starting material to obtain hyperpolarised ¹³C-α-ketoisocaproate by the DNP method.

In a first embodiment, ¹³C-α-ketoisocaproic acid, preferably ¹³C₁-α-ketoisocaproic acid is used as a starting material to obtain hyperpolarised ¹³C-α-ketoisocaproic acid by the DNP method which is then neutralised and converted to hyperpolarised ¹³C-α-ketoisocaproate with the help of a base. ¹³C-α-ketoisocaproic acid is a commercially available compound; alternatively ¹³C-α-ketoisocaproic acid may be prepared from commercially available sodium ¹³C-α-ketoisocaproate by conversion with an acid, a process which is well known in the art and illustrated in the Example part of this application.

In a second embodiment, ¹³C-α-ketoisocaproate, preferably ¹³C₁-α-ketoisocaproate is used as a starting material to obtain hyperpolarised ¹³C-α-ketoisocaproate by the DNP method. Suitable ¹³C-α-ketoisocaproates are for instance sodium ¹³C-α-ketoisocaproate or ¹³C-α-ketoisocaproates which comprise an inorganic cation from the group consisting of NH₄⁺, K⁺, Rb⁺, Cs⁺, Ca²⁺, Sr²⁺ and Ba²⁺, preferably NH₄⁺, K⁺, Rb⁺ or Cs⁺, more preferably K⁺, Rb⁺, Cs⁺ and most preferably Cs⁺, as in detail described in WO-A-2007/111515. In another embodiment, ¹³C-α-ketoisocaproates of an organic amine or amino compound are used as a starting material, more preferably TRIS-¹³C-α-ketoisocaproate or meglumine-¹³C-α-ketoisocaproate acid. These salts are in detail described in WO-A-2007/069909.

The term "TRIS" denotes 2-amino-2-hydroxymethyl-1,3-propanediol and the term "TRIS-¹³C-α-ketoisocaproate" denotes a salt which contains a ¹³C-α-ketoisocaproate anion and a TRIS cation, i.e. TRIS ammonium (2-hydroxymethyl-1,3-propanedioyl ammonium).

For the hyperpolarisation of ¹³C-α-ketoisocaproic acid/¹³C-α-ketoisocaproate by the DNP method, a composition is prepared which comprises ¹³C-α-ketoisocaproic acid or ¹³C-α-ketoisocaproate and a DNP agent.

The DNP agent plays a decisive role in the DNP process as its choice has a major impact on the level of polarisation that can be achieved in ¹³C-α-ketoisocaproic acid/¹³C-α-ketoisocaproate. A variety of DNP agents - in WO-A-99/35508 denoted "OMRI contrast agents" - is known. The use of oxygen-based, sulphur-based or carbon-based stable trityl radicals as described in WO-A-99/35508, WO-A-88/10419, WO-A-90/00904, WO-A-91/12024, WO-A-93/02711 or WO-A-96/39367 has resulted in high levels of polarisation in a variety of different samples.

In a preferred embodiment, the hyperpolarised ¹³C-α-ketoisocaproic acid/¹³C-α-ketoisocaproate is obtained by DNP and the DNP agent used is a trityl radical of formula (1). As briefly mentioned above, the large electron spin polarisation of the DNP agent, i.e. trityl radical is converted to nuclear spin polarisation of ¹³C nuclei in ¹³C-α-ketoisocaproic acid or ¹³C-α-ketoisocaproate via microwave irradiation close to the electron Larmor frequency. The microwaves stimulate communication between electron and nuclear spin systems via e-e and e-n transitions. For effective DNP, i.e. to achieve a high level of polarisation in ¹³C-α-ketoisocaproic acid/¹³C-α-ketoisocaproate, the trityl radical has to be stable and soluble in these compounds or solutions thereof to achieve said intimate contact between ¹³C-α-ketoisocaproic acid/¹³C-α-ketoisocaproate and the trityl radical which is necessary for the aforementioned communication between electron and nuclear spin systems.

In a preferred embodiment, the trityl radical is a radical of the formula (1) 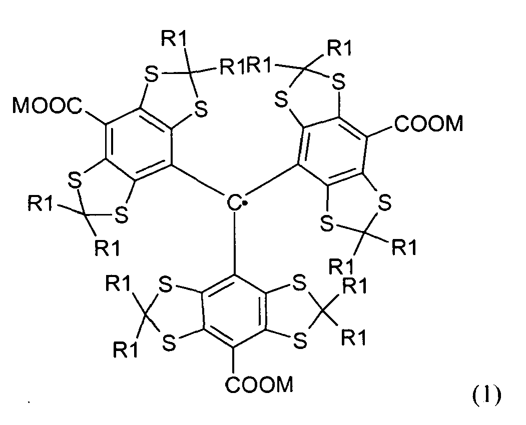 wherein
- M: represents hydrogen or one equivalent of a cation; and
- R1: which is the same or different represents a straight chain or branched C₁-C₆-alkyl group optionally substituted by one or more hydroxyl groups or a group -(CH₂)ₙ-X-R2,
wherein n is 1, 2 or 3;
X is O or S; and
R2 is a straight chain or branched C₁-C₄-alkyl group, optionally substituted by one or more hydroxyl groups.

In a preferred embodiment, M represents hydrogen or one equivalent of a physiologically tolerable cation. The term "physiologically tolerable cation" denotes a cation that is tolerated by the human or non-human animal living body. Preferably, M represents hydrogen or an alkali cation, an ammonium ion or an organic amine ion, for instance meglumine. Most preferably, M represents hydrogen or sodium.

If ¹³C-α-ketoisocaproate is used as a starting material to obtain hyperpolarised ¹³C-a-ketoisocaproate by the DNP method, R1 is preferably the same, more preferably a straight chain or branched C₁-C₄-alkyl group, most preferably methyl, ethyl or isopropyl; or R1 is preferably the same, more preferably a straight chain or branched C₁-C₄-alkyl group which is substituted by one hydroxyl group, most preferably - CH₂-CH₂-OH; or R1 is preferably the same and represents -CH₂-OC₂H₄OH.

If ¹³C-α-ketoisocaproic acid is used as a starting material to obtain hyperpolarised ¹³C-α-ketoisocaproate by the DNP method, R1 is the same or different, preferably the same and preferably represents -CH₂-OCH₃, -CH₂-OC₂H₅, -CH₂-CH₂-OCH-₃,-CH₂-SCH₃, -CH₂-SC₂H₅ or -CH₂-CH₂-SCH₃, most preferably -CH₂-CH₂-OCH₃.

The aforementioned trityl radicals of formula (1) may be synthesized as described in detail in WO-A-88/10419, WO-A-90/00904, WO-A-91/12024, WO-A-93/02711, WO-A-96/39367, WO-A-97/09633, WO-A-98/39277 and WO-A-2006/011811.

Generally, for the DNP process, a solution of the starting material, i.e. ¹³C-α-ketoisocaproic acid or ¹³C-α-ketoisocaproate (in the following denoted "sample") and the DNP agent, preferably a trityl radical, more preferably a trityl radical of formula (1) is prepared. A solvent or a solvent mixture needs to be used to promote dissolution of the DNP agent and the sample. If the hyperpolarised ¹³C-α-ketoisocaproate is intended to be used as an imaging agent for *in vivo* ¹³C-MR detection, it is preferred to keep the amount of solvent to a minimum. To be used as an *in vivo* imaging agent, the hyperpolarised ¹³C-α-ketoisocaproate is usually administered in relatively high concentrations, i.e. a highly concentrated sample is preferably used in the DNP process and hence the amount of solvent is preferably kept to a minimum. In this context, it is also important to mention that the mass of the composition containing the sample, i.e. DNP agent, sample and if necessary solvent, is kept as small as possible. A high mass will have a negative impact on the efficiency of the dissolution process, if dissolution is used to convert the solid composition containing the hyperpolarised ¹³C-α-ketoisocaproic acid/¹³C-α-ketoisocaproate after the DNP process into the liquid state, e.g. for using it as an imaging agent for ¹³C-MR detection. This is due to the fact that for a given volume of dissolution medium in the dissolution process, the mass of the composition to dissolution medium ratio decreases, when the mass of the composition increases. Further, using certain solvents may require their removal before the hyperpolarised ¹³C-α-ketoisocaproate used in the imaging medium of the invention is administered to a human or non-human animal being since they might not be physiologically tolerable.

If ¹³C-α-ketoisocaproic acid is used to obtain hyperpolarised ¹³C-α-ketoisocaproate via DNP, a solution of the DNP agent, preferably a trityl radical and more preferably a trityl radical of formula (1) in ¹³C-α-ketoisocaproic acid, which is a liquid at room temperature, is prepared. A glass former like for instance glycerol or glycol may optionally be added. Intimate mixing of the compounds can be promoted by several means known in the art, such as stirring, vortexing (whirl-mixing) or sonication and/or gentle heating.

If a ¹³C-α-ketoisocaproate like for instance TRIS-¹³C-α-ketoisocaproate is used as the starting material, it may be dissolved in a suitable solvent, preferably water, or solvent mixture and the DNP agent may be added to this solution. In another embodiment, the DNP agent is dissolved in a suitable solvent or solvent mixture and the ¹³C-α-ketoisocaproate is added to this solution. A glass former like for instance glycerol or glycol may optionally be added, for instance if sodium ¹³C-α-ketoisocaproate. Again intimate mixing of the compounds can be promoted by several means known in the art, such as stirring, vortexing or sonication and/or gentle heating.

If ¹³C-α-ketoisocaproates are used as a starting material which crystallize upon freezing, like for instance sodium ¹³C-α-ketoisocaproate, the addition of a glass former to the solvent or solvent mixture is preferred. A suitable solvent for ¹³C-α-ketoisocaproates is water, suitable glass formers are for instance glycol or glycerol. Thus in one embodiment ¹³C-α-ketoisocaproate is dissolved in a solvent or solvent mixture and the DNP agent and a glass former are added to this solution. In another embodiment, the DNP agent is dissolved in a solvent or solvent mixture and the ¹³C-α-ketoisocaproate and a glass former are added to this solution. In yet another embodiment the DNP agent is dissolved in a glass former and the ¹³C-α-ketoisocaproate and a solvent or solvent mixture is added to this solution.

The composition to be DNP-polarised comprising ¹³C-α-ketoisocaproic acid or ¹³C-α-ketoisocaproate and a DNP agent may further comprise a paramagnetic metal ion. It has been found that the presence of paramagnetic metal ions may result in increased polarisation levels in the compound to be polarised by DNP as described in detail in WO-A2-2007/064226.

The term "paramagnetic metal ion" denotes paramagnetic metal ions in the form of their salts or in chelated form, i.e. paramagnetic chelates. The latter are chemical entities comprising a chelator and a paramagnetic metal ion, wherein said paramagnetic metal ion and said chelator form a complex, i.e. a paramagnetic chelate.

In a preferred embodiment, the paramagnetic metal ion is a salt or paramagnetic chelate comprising Gd³⁺, preferably a paramagnetic chelate comprising Gd³⁺. In a more preferred embodiment, said paramagnetic metal ion is soluble and stable in the composition to be polarised.

As with the DNP agent described before, the ¹³C-α-ketoisocaproic acid/¹³C-α-ketoisocaproate to be polarised must be in intimate contact with the paramagnetic metal ion as well. The composition used for DNP comprising ¹³C-α-ketoisocaproic acid or ¹³C-α-ketoisocaproate, a DNP agent and a paramagnetic metal ion may be obtained in several ways.

If ¹³C-α-ketoisocaproic acid is used as a starting material, it is preferred to add the DNP agent to ¹³C-α-ketoisocaproic acid, either as a solid or in solution. If the trityl radical of formula (1) is used as DNP agent, it is preferably added as a solid. In a subsequent step, the paramagnetic metal ion is added. The paramagnetic metal ion might be added as a solid or in solution. Alternatively, a solution of the DNP agent and paramagnetic metal ion may be prepared and ¹³C-α-ketoisocaproic acid is added to this solution or the solution is added to ¹³C-α-ketoisocaproic acid. Intimate mixing of the compounds can be promoted by several means known in the art, such as stirring, vortexing or sonication and/or gentle heating.

If a ¹³C-α-ketoisocaproate like TRIS-¹³C-α-ketoisocaproate is used as the starting material, it may be dissolved in a suitable solvent or solvent mixture and the DNP agent may be added to this solution. The DNP agent, preferably a trityl radical, might be added as a solid or in solution. In a subsequent step, the paramagnetic metal ion is added. Also the paramagnetic metal ion might be added as a solid or in solution. In another embodiment, the DNP agent and the paramagnetic metal ion are dissolved in suitable solvents or a suitable solvent and to this solution is added the ¹³C-α-ketoisocaproate. In yet another embodiment, the DNP agent (or the paramagnetic metal ion) is dissolved in a suitable solvent and added to the solid or dissolved ¹³C-α-ketoisocaproate. In a subsequent step the paramagnetic metal ion (or the DNP agent) is added to this solution, either as a solid or in solution. Preferably, the amount of solvent to dissolve all the compounds is kept to a minimum. As discussed before, if ¹³C-α-ketoisocaproates are used as a starting material which crystallize upon freezing, like for instance sodium ¹³C-α-ketoisocaproate, the addition of a glass former to the solvent or solvent mixture is preferred. Again intimate mixing of the compounds can be promoted by several means known in the art, such as stirring, vortexing or sonication and/or gentle heating.

If a trityl radical is used as DNP agent, a suitable concentration of such a trityl radical is I to 25 mM, preferably 2 to 20 mM, more preferably 10 to 15 mM in the composition used for DNP. If a paramagnetic metal ion is added to the composition, a suitable concentration of such a paramagnetic metal ion is 0.1 to 6 mM (metal ion) in the composition, and a concentration of 0.3 to 4 mM is preferred.

After having prepared a composition comprising ¹³C-α-ketoisocaproic acid or ¹³C-α-ketoisocaproate, the DNP agent and optionally a paramagnetic metal ion said composition is frozen by methods known in the art, e.g. by freezing it in a freezer, in liquid nitrogen or by simply placing it in the DNP polariser, where liquid helium will freeze it. In a preferred embodiment, the composition is frozen as "beads" before it is inserted into to polariser. Such beads may be obtained by adding the composition drop wise to liquid nitrogen. A more efficient dissolution of such beads has been observed, which is especially relevant if larger amounts of ¹³C-α-ketoisocaproic acid or ¹³C-α-ketoisocaproate are polarised, for instance when the compound is intended to be used in an *in vivo* ¹³C-MR detection method.

If a paramagnetic metal ion is present in the composition said composition may be degassed before freezing, e.g. by bubbling helium gas through the composition (e.g. for a time period of 2 - 15 min) but degassing can be effected by other known common methods.

The DNP technique is for instance described in WO-A-98/58272 and in WO-A-01/96895. Generally, a moderate or high magnetic field and a very low temperature are used in the DNP process, e.g. by carrying out the DNP process in liquid helium and a magnetic field of about 1 T or above. Alternatively, a moderate magnetic field and any temperature at which sufficient polarisation enhancement is achieved may be employed. In a preferred embodiment, the DNP process is carried out in liquid helium and a magnetic field of about 1 T or above. Suitable polarisation units are for instance described in WO-A-02/37132. In a preferred embodiment, the polarisation unit comprises a cryostat and polarising means, e.g. a microwave chamber connected by a wave guide to a microwave source in a central bore surrounded by magnetic field producing means such as a superconducting magnet. The bore extends vertically down to at least the level of a region P near the superconducting magnet where the magnetic field strength is sufficiently high, e.g. between I and 25 T, for polarisation of the sample nuclei to take place. The bore for the probe (i.e. the frozen composition to be polarised) is preferably sealable and can be evacuated to low pressures, e.g. pressures in the order of 1 mbar or less. A probe introducing means such as a removable transporting tube can be contained inside the bore and this tube can be inserted from the top of the bore down to a position inside the microwave chamber in region P. Region P is cooled by liquid helium to a temperature low enough to for polarisation to take place, preferably temperatures of the order of 0.1 to 100 K, more preferably 0.5 to 10 K, most preferably I to 5 K. The probe introducing means is preferably sealable at its upper end in any suitable way to retain the partial vacuum in the bore. A probe-retaining container, such as a probe-retaining cup, can be removably fitted inside the lower end of the probe introducing means. The probe-retaining container is preferably made of a light-weight material with a low specific heat capacity and good cryogenic properties such, e.g. KelF (polychlorotrifluoro-ethylene) or PEEK (polyetheretherketone) and it may be designed in such a way that it can hold more than one probe.

The probe is inserted into the probe-retaining container, submerged in the liquid helium and irradiated with microwaves, preferably at a frequency of about 94 GHz at 200 mW. The level of polarisation may be monitored by for instance acquiring solid state ¹³C-NMR signals of the probe during microwave irradiation. Generally, a saturation curve is obtained in a graph showing NMR signal vs. time. Hence it is possible to determine when the optimal and/or sufficient polarisation level is reached. A solid state ¹³C-NMR measurement suitably consists of a simple pulse-acquire NMR sequence using a low flip angle. The signal intensity of the dynamic nuclear polarised nuclei, i.e. ¹³C nuclei in ¹³C-α-ketoisocaproic acid or ¹³C-α-ketoisocaproate is compared with the signal intensity of the ¹³C nuclei in ¹³C-α-ketoisocaproic acid or ¹³C-α-ketoisocaproate before DNP. The polarisation is then calculated from the ratio of the signal intensities before and after DNP.

After the DNP process, the solid composition comprising the hyperpolarised ¹³C-α-ketoisocaproic acid or ¹³C-α-ketoisocaproate is transferred from a solid state to a liquid state, i.e. liquefied. This can be done by dissolution in an appropriate solvent or solvent mixture (dissolution medium) or by melting the solid composition. Dissolution is preferred and the dissolution process and suitable devices therefore are described in detail in WO-A-02/37132. The melting process and suitable devices for the melting are for instance described in WO-A-02/36005. Briefly, a dissolution unit/melting unit is used which is either physically separated from the polariser or is a part of an apparatus that contains the polariser and the dissolution unit/melting unit. In a preferred embodiment, dissolution/melting is carried out at an elevated magnetic field, e.g. inside the polariser, to improve the relaxation and retain a maximum of the hyperpolarisation. Field nodes should be avoided and low field may lead to enhanced relaxation despite the above measures.

If ¹³C-α-ketoisocaproate has been used as the starting material for the dynamic nuclear polarisation and if the solid composition comprising the hyperpolarised ¹³C-α-ketoisocaproate is liquefied by dissolution, an aqueous carrier, preferably a physiologically tolerable and pharmaceutically accepted aqueous carrier like water, a buffer solution or saline is suitably used as a solvent, especially preferably if the hyperpolarised ¹³C-α-ketoisocaproate is intended for use in an imaging medium for *in vivo* ¹³C-MR detection. The aqueous carrier may contain a base to adjust the pH of the final solution in such a way that it is suitable for *in vivo* administration. Suitable pH ranges from 6.8 to 7.8. For *in vitro* applications also non aqueous solvents or solvent mixtures may be used, for instance DMSO or methanol or mixtures comprising an aqueous carrier and a non aqueous solvent, for instance mixtures of DMSO and water or methanol and water. In another preferred embodiment, the aqueous carrier or the non aqueous solvents or solvent mixtures may further comprise one or more compounds which are able to bind or complex free paramagnetic ions, e.g. chelating agents like DTPA or EDTA.

If ¹³C-α-ketoisocaproic acid has been used as the starting material for the dynamic nuclear polarisation, the hyperpolarised ¹³C-α-ketoisocaproic acid obtained needs to be converted to ¹³C-α-ketoisocaproate. If the solid composition comprising the hyperpolarised ¹³C-a-ketoisocaproic acid is liquefied by dissolution, and the hyperpolarised ¹³C-α-ketoisocaproate is intended to be used *in vivo,* the dissolution medium is preferably an aqueous carrier, e.g. water or a buffer solution, preferably a physiologically tolerable buffer solution or it comprises an aqueous carrier, e.g. water or a buffer solution, preferably a physiologically tolerable buffer solution. The terms "buffer solution" and "buffer" are hereinafter used interchangeably. In the context of this application "buffer" denotes one or more buffers, i.e. also mixtures of buffers.

Preferred buffers are physiologically tolerable buffers, more preferably buffers which buffer in the range of about pH 7 to 8 like for instance phosphate buffer (KH₂PO₄/Na₂HPO₄), ACES, PIPES, imidazole/HCl, BES, MOPS, HEPES, TES, TRIS, HEPPS or TRICIN.

To convert hyperpolarised ¹³C-α-ketoisocaproic acid into hyperpolarised ¹³C-α-ketoisocaproate, ¹³C-α-ketoisocaproic acid is generally reacted with a base. In one embodiment, ¹²C-α-ketoisocaproic acid is reacted with a base to convert it to ¹³C-α-ketoisocaproate. For *in vivo* intended use an aqueous carrier is subsequently added. In another preferred embodiment the aqueous carrier and the base are combined in one solution and this solution is added to ¹³C-α-ketoisocaproic acid, dissolving it and converting it into ¹³C-α-ketoisocaproate at the same time. In a preferred embodiment, the base is an aqueous solution of NaOH, Na₂CO₃ or NaHCO₃, most preferred the base is NaOH.

In another preferred embodiment, the aqueous carrier buffer or - where applicable - the combined aqueous carrier/base solution further comprises one or more compounds which are able to bind or complex free paramagnetic ions, e.g. chelating agents like DTPA or EDTA.

For *in vitro* applications of hyperpolarised ¹³C-α-ketoisocaproate also non aqueous solvents or solvent mixtures may be used, for instance DMSO or methanol or mixtures comprising an aqueous carrier and a non aqueous solvent, for instance mixtures of DM SO and water or methanol and water.

If hyperpolarisation is carried out by the DNP method, the DNP agent, preferably a trityl radical and the optional paramagnetic metal ion may be removed from the liquid containing the hyperpolarised ¹³C-α-ketoisocaproic acid or ¹³C-α-ketoisocaproate. Removal of these compounds is preferred if the hyperpolarised ¹³C-α-ketoisocaproic acid or ¹³C-α-ketoisocaproate is intended for use in an imaging medium for *in vivo* use. If hyperpolarised ¹³C-α-ketoisocaproic acid was used as a starting material for DNP, it is preferred to first convert the hyperpolarised ¹³C-α-ketoisocaproic acid into ¹³C-α-ketoisocaproate and remove the DNP agent and the optional paramagnetic metal ion after said conversion has taken place.

Methods which are useful to remove the trityl radical and the paramagnetic metal ion are known in the art and described in detail in WO-A2-2007/064226 and WO-Al-2006/011809.

In a preferred embodiment the hyperpolarised ¹³C-α-ketoisocaproate of the imaging medium according to the invention is obtained by dynamic nuclear polarisation of a composition that comprises ¹³C-α-ketoisocaproic acid or TRIS-¹³C-α-ketoisocaproate or sodium ¹²C-α-ketoisocaproate, a trityl radical of formula (1) and optionally a paramagnetic chelate comprising Gd³⁺.

The imaging medium according to the invention may be used as imaging medium for *in vitro* ¹³C-MR detection, e.g. ¹³C-MR detection of cell cultures, samples, ex *vivo* tissue or isolated organs derived from the human or non-human animal body. For this purpose, the imaging medium is provided as a composition that is suitable for being added to, for instance, cell cultures, samples like urine, blood or saliva, *ex vivo* tissues like biopsy tissues or isolated organs. Such an imaging medium preferably comprises in addition to the imaging agent, i.e. hyperpolarised ¹³C-α-ketoisocaproate, a solvent which is compatible with and used for *in vitro* cell or tissue assays, for instance DMSO or methanol or solvent mixtures comprising an aqueous carrier and a non aqueous solvent, for instance mixtures of DMSO and water or a buffer solution or methanol and water or a buffer solution. As it is apparent for the skilled person, pharmaceutically acceptable carriers, excipients and formulation aids may be present in such an imaging medium but are not required for such a purpose.

Further, the imaging medium according to the invention may be used as imaging medium for *in vivo* ¹³C-MR detection, i.e. ¹³C-MR detection carried out on living human or non-human animal beings. For this purpose, the imaging medium needs to be suitable for administration to a living human or non-human animal body. Hence such an imaging medium preferably comprises in addition to the imaging agent, i.e. hyperpolarised ¹³C-α-ketoisocaproate, an aqueous carrier, preferably a physiologically tolerable and pharmaceutically accepted aqueous carrier like water, a buffer solution or saline. Such an imaging medium may further comprise conventional pharmaceutical or veterinary carriers or excipients, e.g. formulation aids such as stabilizers, osmolality adjusting agents, solubilising agents and the like which are conventional for diagnostic compositions in human or veterinary medicine.

If the imaging medium of the invention is used for *in vivo* ¹³C-MR detection, i.e. in a living human or non-human animal body, said imaging medium is preferably administered to said body parenterally, preferably intravenously. Generally, the body under examination is positioned in an MR magnet. Dedicated ¹³C-MR RF-coils are positioned to cover the area of interest. Exact dosage and concentration of the imaging medium will depend upon a range of factors such as toxicity and the administration route. Generally, the imaging medium is administered in a concentration of up to 1 mmol ¹³C-α-ketoisocaproate per kg bodyweight, preferably 0.01 to 0.5 mmol/kg, more preferably 0.1 to 0.3 mmol/kg. At less than 400 s after the administration, preferably less than 120 s, more preferably less than 60 s after the administration, especially preferably 20 to 50 s an MR imaging sequence is applied that encodes the volume of interest in a combined frequency and spatial selective way. The exact time of applying an MR sequence is highly dependent on the volume of interest.

The imaging medium according to the invention is preferably used in a method of ¹³C-MR detection.

In a second aspect the invention provides an imaging medium comprising hyperpolarised ¹³C-α-ketoisocaproate for use in a method of ¹³C-MR detection,

In a preferred first embodiment, the invention provides a method of ¹³C-MR detection using an imaging medium comprising hyperpolarised ¹³C-α-ketoisocaproate wherein signals of ¹³C-leucine and optionally of ¹³C-α-ketoisocaproate are detected.

In a preferred second embodiment, the invention provides a method of ¹³C-MR detection using an imaging medium comprising hyperpolarised ¹³C-α-ketoisocaproate wherein signals of ¹³CO₂ and/or ¹³C-bicarbonate and optionally of ¹³C-α-ketoisocaproate are detected.

In a preferred third embodiment, the invention provides a method of ¹³C-MR detection using an imaging medium comprising hyperpolarised ¹³C-α-ketoisocaproate wherein signals of ¹³C-leucine and ¹³CO₂ and/or ¹³C-bicarbonate and optionally of ¹³C-α-ketoisocaproate are detected.

The term "signals of ¹³C-leucine and optionally ¹³C-α-ketoisocaproate are detected" means that in the method of the invention, only the signal of ¹³C-leucine is detected or the signals of ¹³C-leucine and ¹³C-α-ketoisocaproate are detected.

The term "signals of ¹³CO₂ and/or ¹³C-bicarbonate and optionally of ¹³C-α-ketoisocaproate are detected" means that in the method of the invention only the signal of ¹³CO₂ or only the signal of ¹³C-bicarbonate is detected or that the signals of ¹³CO_{Z} and ¹³C-bicarbonate are detected or that the signals of ¹³CO₂ and ¹³C-α-ketoisocaproate or the signals of ¹³C-bicarbonate and ¹³C-α-ketoisocaproate or the signals of are detected or that the signals of CO₂ and ¹³C-bicarbonate and ¹³C-α-ketoisocaproate are detected.

The term "signals of ¹³C-leucine and ¹³CO₂ and/or ¹³C-bicarbonate and optionally of ¹³C-α-ketoisocaproate are detected" means that in the method of the invention the signals of ¹³C-leucine and ¹³CO₂ or the signals of ¹³C-leucine and ¹³C-bicarbonate or the signals of ¹³C-leucine and ¹³CO₂ and ¹³C-bicarbonate are detected. It further means that the signals of ¹³C-leucine and ¹³CO₂ and ¹³C-α-ketoisocaproate or the signals of ¹³C-leucine and ¹³C-bicarbonate and ¹³C-α-ketoisocaproate or the signals of ¹³C-leucine and ¹³CO₂ and ¹³C-bicarbonate and ¹³C-α-ketoisocaproate are detected

The term "¹³C-leucine" denotes 2-amino-4-methyl-pentanoic acid that is isotopically enriched with ¹³C, i.e. in which the amount of ¹³C isotope is greater than its natural abundance. Unless otherwise specified, the term "¹³C-leucine" denotes a compound which is ¹³C-enriched at the C1-and/or C2-and/or C4-position The position of the isotopic enrichment in ¹³C-leucine is of course dependent on the position of the isotopic enrichment in its parent compound ¹³C-α-ketoisocaproate. Thus, if for example hyperpolarised ¹³C₁-α-ketoisocaproate was used in the imaging medium used in the method of the invention, the signal of ¹³C₁-leucine is detected.

The term "¹³C-bicarbonate" denotes HCO₃⁻ that is isotopically enriched with ¹³C, i.e. in which the amount of ¹³C isotope is greater than its natural abundance. Likewise the term "¹³CO₂" denotes carbon dioxide that is isotopically enriched with ¹³C, i.e. in which the amount of ¹³C isotope is greater than its natural abundance. Whether it is possible to detect ¹³C-bicarbonate and/or ¹³CO₂ is of course dependent on the position of the isotopic enrichment in its parent compound ¹³C-α-ketoisocaproate. Only if ¹³C-α-ketoisocaproate which is ¹³C-enriched at the C1-position is used in the imaging medium used in the method of the invention, ¹³C-bicarbonate and ¹³CO₂ is formed and thus may be detected by ¹³C-MR detection.

The metabolic conversion of α-ketoisocaproate acid to leucine and carbon dioxide is shown in scheme I for ¹³C₁-α-ketoisocaproate; * denotes the ¹³C-label ¹³C-α-ketoisocaproate is converted to ¹³C₁-leucine by branched chain aminotransferase (BCAT, EC 2.6.1.42) and to ¹³CO₂ (and subsequently ¹³C-bicarbonate) by branched chain alpha-keto acid dehydrogenase (BCKD, EC 1.2.4.4.).

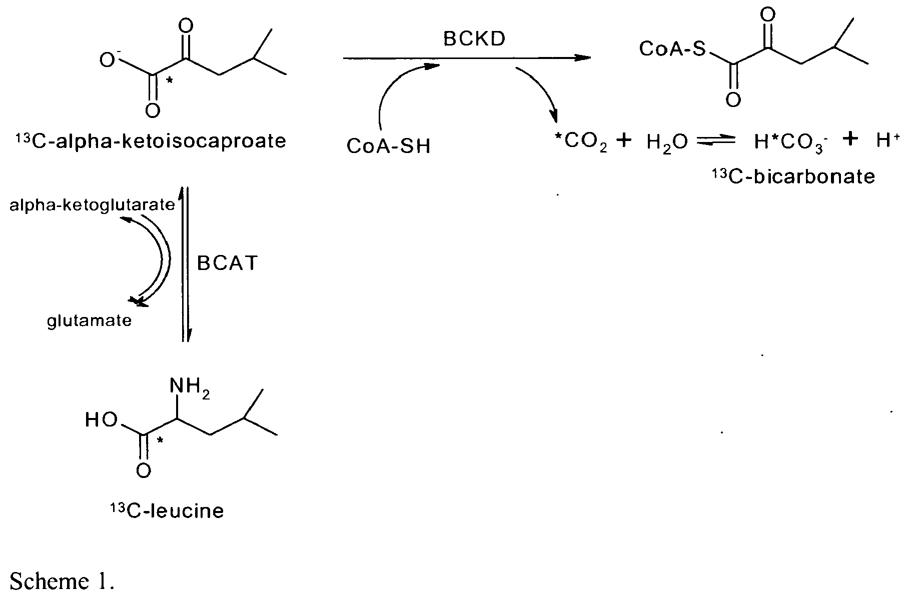

The term "signal" in the context of the invention refers to the MR signal amplitude or integral or peak area to noise of peaks in a ¹³C-MR spectrum which represent ¹³C-leucine, ¹³CO₂, ¹³C-bicarbonate and/or ¹³C-α-ketoisocaproate. In a preferred embodiment, the signal is the peak area.

In a preferred embodiment the above-mentioned signals of ¹C-leucine, ¹³CO₂, ¹³C-bicarbonate and/or ¹³C-α-ketoisocaproate are used to generate a metabolic profile of a living human or non-human animal being. Said metabolic profile may be derived from the whole body, e.g. obtained by whole body *in vivo* ¹³C-MR detection. Alternatively, said metabolic profile is generated from a region or volume of interest, i.e. a certain tissue, organ or part of said human or non-human animal body.

In another preferred embodiment the above-mentioned signals of ¹³C-leucine, ¹³CO₂, ¹³C-bicarbonate and/or ¹³C-α-ketoisocaproate are used to generate a metabolic profile of cells in a cell culture, of samples like urine, blood or saliva, of ex *vivo* tissue like biopsy tissue or of an isolated organ derived from a human or non-human animal being. Said metabolic profile is then generated by *in vitro ¹³C-MR* detection.

Thus, in a preferred first embodiment, the invention provides an imaging medium comprising hyperpolarised ¹³C-α- ketoisocaproate for use in a method of ¹³C-MR detection, wherein signals of ¹³C-leucine and optionally of ¹³C-α-ketoisocaproate are detected and wherein said signals are used to generate a metabolic profile.

In a preferred second embodiment, the invention provides an imaging medium comprising hyperpolarised ¹³C-α- ketoisocaproate for use in a method of ¹³C-MR detection, wherein signals of ¹³CO₂ and/or ¹³C-bicarbonate and optionally of ¹³C-α-ketoisocaproate are detected and wherein said signals are used to generate a metabolic profile.

In a preferred third embodiment, the invention provides for use in a method of ¹³C-MR detection, an imaging medium comprising hyperpolarised ¹³C-α-ketoisocaproate wherein signals of ¹³C-leucine and ¹³CO₂ and/or ¹³C-bicarbonate and optionally of ¹³C-α-ketoisocaproate are detected and wherein said signals are used to generate a metabolic profile.

In a more preferred first embodiment, the signals of ¹³C-leucine and ¹³C-a-ketoisocaproate are used to generate said metabolic profile.

In one embodiment, the spectral signal intensities of ¹³C-leucine and optional ' uC-α-ketoisocaproate are used to generate the metabolic profile. In another embodiment, the spectral signal integrals of ¹³C-leucine and optionally ¹³C-α-ketoisocaproate are used to generate the metabolic profile. In another embodiment, signal intensities from separate images of ¹³C-leucine and ¹³C-α-ketoisocaproate are used to generate the metabolic profile. In yet another embodiment, the signal intensities of ¹³C-leucine and optionally ¹³C-α-ketoisocaproate are obtained at two or more time points to calculate the rate of change of ¹³C-leucine and optionally the rate of change of ¹³C-α-ketoisocaproate.

In another embodiment the metabolic profile includes or is generated using processed signal data of ¹³C-leucine and optionally ¹³C-α-ketoisocaproate, e.g. ratios of signals, corrected signals, or dynamic or metabolic rate constant information deduced from the signal pattern of multiple MR detections, i.e. spectra or images.

Hence, in a preferred embodiment a corrected ¹³C-leucine signal, i.e. ¹³C-leucine to ¹³C-α-ketoisocaproate signal is included into or used to generate the metabolic profile. In a further preferred embodiment, a corrected ¹³C-leucine to total ¹³C-carbon signal is included into or used to generate the metabolic profile with total ¹³C-carbon signal being the sum of the signals of ¹³C-leucine and ¹³C-α-ketoisocaproate. In a more preferred embodiment, the ratio of ¹³C-leucine to ¹³C-α-ketoisocaproate is included into or used to generate the metabolic profile.

In a more preferred second embodiment, the signals of CO₂ and/or ¹³C-bicarbonate and ¹³C-α-ketoisocaproate are used to generate said metabolic profile.

In one embodiment, the spectral signal intensities of CO₂ and/or ¹³C-α-bicarbonate and optionally ¹³C-α-ketoisocaproate are used to generate the metabolic profile. In another embodiment, the spectral signal integrals of ¹³CO₂ and/or ¹³C-bicarbonate and optionally ¹³C-α-ketoisocaproate are used to generate the metabolic profile. In another embodiment, signal intensities from separate images of ¹³CO₂ and ¹³C-bicarbonate and optionally ¹³C-α-ketoisocaproate or separate images of ¹³CO₂ and ¹³C-α-ketoisocaproate or separate images of ¹³C-bicarbonate and ¹³C-α-ketoisocaproate are used to generate the metabolic profile. In yet another embodiment, the signal intensities of ¹³CO₂ and/or ¹³C-bicarbonate and optionally of ¹³C-α-ketoisocaproate are obtained at two or more time points to calculate the rate of change of ¹³CO₂ and/or ¹³C-bicarbonate and optionally the rate of change of ¹³C-α-ketoisocaproate.

In another embodiment the metabolic profile includes or is generated using processed signal data of ¹³CO₂ and/or ¹³C-bicarbonate and optionally ¹³C-α ketoisocaproate, e.g. ratios of signals, corrected signals, or dynamic or metabolic rate constant information deduced from the signal pattern of multiple MR detections, i.e. spectra or images.

Hence, in a preferred embodiment a corrected ¹³CO₂ and/or ¹³C-bicarbonate signal, i.e. ¹³CO₂ to ¹³C-α-ketoisocaproate signal or ¹³C-bicarbonate to ¹³C-α-ketoisocaproate is included into or used to generate the metabolic profile. In a further preferred embodiment, a corrected ¹³CO₂ and/or ¹³C-bicarbonate to total ¹³C-carbon signal is included into or used to generate the metabolic profile with total ¹³C-carbon signal being the sum of the signals of ¹³CO₂ and/or ¹³C-bicarbonate signal and ¹³C-α-ketoisocaproate. In a more preferred embodiment, the ratio of ¹³CO₂ and/or ¹³C-bicarbonate signal to ¹³C-α-ketoisocaproate signal is included into or used to generate the metabolic profile.

In a more preferred third embodiment, the signals of ¹³C-C-leucine and ¹³CO₂ and/or ¹³C-bicarbonate and optionally of ¹³C-α-ketoisocaproate are used to generate a metabolic profile.

In one embodiment, the spectral signal intensities of ¹³C-leucine and ¹³CO₂ and/or ¹³C-α-bicarbonate and optionally ¹³C-α-ketoisocaproate are used to generate the metabolic profile. In another embodiment, the spectral signal integrals of ¹³C-leucine and ¹³CO₂ and/or ¹³C-bicarbonate and optionally ¹³C-α-ketoisocaproate are used to generate the metabolic profile. In another embodiment, signal intensities from separate images of ¹³C-leucine and ¹³CO₂ and ¹³C-bicarbonate and optionally ¹³C-α ketoisocaproate or separate images of ¹³C-leucine and ¹³CO₂ and optionally ¹³C-α-ketoisocaproate or separate images of ¹³C-leucine and ¹³C-bicarbonate and optionally ¹³C-α-ketoisocaproate are used to generate the metabolic profile. In yet another embodiment, the signal intensities of ¹³C-leucine and ¹³CO₂ and/or ¹³C-bicarbonate and optionally of ¹³C-α-ketoisocaproate are obtained at two or more time points to calculate the rate of change of ¹³C-leucine and ¹³CO₂ and/or ¹³C-bicarbonate and optionally the rate of change of ¹³C-α-ketoisocaproate.

In another embodiment the metabolic profile includes or is generated using processed signal data of ¹³C-leucine and ¹³CO₂ and/or ¹³C-bicarbonate and optionally ¹³C-α-ketoisocaproate, e.g. ratios of signals, corrected signals, or dynamic or metabolic rate constant information deduced from the signal pattern of multiple MR detections, i.e. spectra or images.

Hence, in a preferred embodiment corrected ¹³C-leucine and ¹³CO₂ and/or ¹³C-bicarbonate signals, i.e. ¹³C-leucine to ¹³CO₂ signal or ¹³C-leucine to ¹³C-bicarbonate signal and optionally ¹³C-leucine to ¹³C-α-ketoisocaproate signal is included into or used to generate the metabolic profile. In a further preferred embodiment, a corrected C-leucine and ¹³CO₂ and/or ¹³C-bicarbonate to total ¹³C-carbon signal is included into or used to generate the metabolic profile with total ¹³C-carbon signal being the sum of the signals of ¹³C-leucine and ¹³CO₂ and/or ¹³C-bicarbonate signal and optionally ¹³C-α-ketoisocaproate. In a more preferred embodiment, the ratio of ¹³C-leucine and ¹³CO₂ and/or ¹³C-bicarbonate signal to ¹³C-α-ketoisocaproate signal is included into or used to generate the metabolic profile

The metabolic profile generated provides information about the metabolic activity of the body, part of the body, cells, tissue, body sample etc under examination and said information may be used in a subsequent step for, e.g. identifying diseases.

Such a disease is preferably cancer since tumour tissue is usually characterized by a higher mctabolic activity than healthy tissue. Such a higher metabolic activity would be apparent from comparing the metabolic profile of a tumour or of an *ex vivo* sample of a tumour with the metabolic profile of healthy tissue (e.g. surrounding tissue or healthy ex *vivo* tissue) and may manifest itself in the metabolic profile by high signals of ¹³C-leucine and/or ¹³CO₂ and/or ¹³C-bicarbonate or by a high corrected ¹³C--leucine and/or ¹³CO₂ and/or ¹³C-bicarbonate signal or a high ratio of ¹³C-leucine to ''C-a-ketoisocaproate signal and/or ¹³CO₂ and/or ¹³C-bicarbonate to ¹³C-α-ketoisocaproate signal or a high ratio of ¹³C-leucine signal to total carbon signal and/or a high ratio of ¹³CO₂ and/or ¹³C-bicarbonate signal to total carbon signal or a high metabolic rate of ¹³C-leucine and/or ¹³CO₂ and/or ¹³C-bicarbonate build-up.

In cancer tissue the concentration of glutamate is often higher than in healthy tissue. This co-substrate will enable a high turn over of ¹³C-αketoisocaproate to ¹³C-leucine in the cancer tissue where the BCAT activity is high.

The term "high" is a relative term and it has to be understood that the "high signal, ratio, metabolic rate" etc. which is seen in a metabolic profile of a diseased tissue as described above is increased compared to the signal, ratio, metabolic rate etc. which is seen in a metabolic profile of a healthy tissue.

In hepatic steatosis, there is a lower activity of the enzyme BCKD in the liver and a ¹³C-breath test based on the decarboxylation of ¹³C-α-ketoisocaproate is used for diagnosing said disease state. In this test the exhaled ¹³CO₂ is collected dynamically and quantified by methods in the art. Hence there is an indication that the information provided by the metabolic profile generated in the preferred embodiment of the method according to the invention may be used for identification of liver related diseases like fatty liver, liver fibrosis or liver cirrhosis. For such liver-related diseases, it can be assumed that they would manifest themselves in a metabolic profile of a diseased liver by a change in ¹³CO₂ and/or ¹³C-bicarbonate signal and/or ratio of these metabolites to ¹³C-α-ketoisocaproate when compared to a metabolic profile of a healthy liver.

Yet another disease may be kidney related diseases since it is known that the activity of BCAT which catalyzes the conversion of ¹³C-α-ketoisocaproate to ¹³C-leucine is highly active in the kidneys. In kidney diseases which manifest themselves by a change in BCAT activity, it can be assumed that said change, which may be a change in ¹³C-leucine signal and/or a change in ratio of ¹³C-leucine to ¹³C-α-ketoisocaproate can be detected in a metabolic profile of a diseased kidney/diseased kidney tissue when compared to a metabolic profile of a healthy kidney/healthy surrounding kidney tissue.

Anatomical and/or - where suitable - perfusion information may be included in the method of the invention for identification of diseases. Anatomical information may for instance be obtained by acquiring a proton or ¹³C-MR image with or without employing a suitable contrast agent before or after the method of the invention.

In another preferred embodiment, the imaging medium comprising hyperpolarised ¹³C-α-ketoisocaproate is administered repeatedly, thus allowing dynamic studies. This is a further advantage of the imaging medium comprising hyperpolarised ¹³C-α-ketoisocaproate compared to other MR detection methods using conventional MR contrast agents which - in higher doses -may show toxic effects. α-Ketoisocaproate is present in the human body and hyperpolarised ¹³C-α-ketoisocaproate was well tolerated in the animal models we have used and described in the Examples part of this application. Hence it is expected that hyperpolarised ¹³C-α-ketoisocaproate will be well tolerated in patients as well and thus administration of repeated doses of this compound should be possible.

As stated above, the metabolic profile provides information about the metabolic activity of the body, part of the body, cells, tissue, body sample etc. under examination and said information may be used in a subsequent step for, e.g. identifying diseases. However, a physician may also use this information in a further step to choose the appropriate treatment for the patient under examination.

Thus, said information may be used to monitor treatment response, e.g. treatment success of the above mentioned diseases, and its sensitivity makes the method especially suitable for monitoring early treatment response, i.e. response to treatment shortly after its commencement.

In yet another embodiment, the method of the invention may be used to assess drug efficacy. In said embodiment, potential drugs for curing a certain disease like for instance anti-cancer drugs, may be tested at a very early stage in drug screening, for instance *in vitro* in a cell culture which is a relevant model for said certain disease or in diseased *ex vivo* tissue or a diseased isolated organ. Alternatively, potential drugs for curing a certain disease may be tested at an early stage in drug screening *in vivo,* for instance in an animal model which is relevant for said certain disease. By comparing the metabolic profile of said cell culture, *ex vivo* tissue, isolated or test animal before and after treatment with a potential drug, the efficacy of said drug and thus treatment response and success can be determined which of course provides valuable information in the screening of potential drugs.

Yet another aspect of the invention is a composition comprising ¹³C-α-ketoisocaproic acid or ¹³C-α-ketoisocaproate, a DNP agent according to formula (1) and optionally a paramagnetic metal ion. Said composition can be used for obtaining hyperpolarised ¹³C-α-ketoisocaproate by dynamic nuclear polarisation (DNP) which can be used as imaging agent (MR active agent) in the imaging medium according to the invention.

In one embodiment, the composition according to the invention comprises ¹³C-α-ketoisocaproic acid, a DNP agent wherein the DNP agent is a trityl radical of formula (1) wherein M represents hydrogen or sodium and R1 is the same or different, preferably the same and preferably represents -CH₂-OCH₃, -CH₂-OC₂H₅, -CH₂-CH₂-OCH₃, -CH₂-SCH₃, -CH₂-SC₂H₅ or -CH₂-CH₂-SCH₃, most preferably -CH₂-CH₂-OCH₃ and optionally a paramagnetic metal ion. In another preferred embodiment said composition comprises a paramagnetic metal ion, preferably a salt or paramagnetic chelate comprising Gd³⁺ and more a paramagnetic chelate comprising Gd³⁺. Optionally, said composition further comprises a solvent or solvents and/or a glass former and optionally a paramagnetic metal ion. The composition may comprise a glass former like for instance glycerol. The aforementioned compositions can be used for obtaining hyperpolarised ¹³C-α-ketoisocaproic acid by dynamic nuclear polarisation (DNP) with a high polarisation level. Said hyperpolarised ¹³C-α-ketoisocaproic acid can be converted into hyperpolarised ¹³C-α-ketoisocaproate by dissolution with a base, e.g. NaOH, as described earlier in the application.

Said composition may comprise ¹³C-α-ketoisocaproate, preferably TRIS-¹³C-α-ketoisocaproate or sodium ¹³C-α-ketoisocaproate, a DNP agent according to formula (1) and optionally a paramagnetic metal ion. In a preferred embodiment, said DNP is a (trityl radical of formula (1) wherein M represents hydrogen or sodium and R1 is preferably the same, more preferably a straight chain or branched C₁-C₄-alkyl group, most preferably methyl, ethyl or isopropyl; or R1 is preferably the same, more preferably a straight chain or branched C₁-C₄-alkyl group which is substituted by one hydroxyl group, most preferably -CH₂-CH₂-OH; or R1 is preferably the same and represents -CH₂-OC₂H₄OH. In another preferred embodiment said composition comprises a paramagnetic metal ion, preferably a salt or paramagnetic chelate comprising Gd³⁺ and more a paramagnetic chelate comprising Gd³⁺. Suitably, said composition further comprises a solvent or solvents; preferably an aqueous carrier. Optionally, said composition comprises a glass former like for instance glycerol. The aforementioned compositions can be used for obtaining hyperpolarised ¹³C-α-ketoisocaproate by dynamic nuclear polarisation (DNP) with a high polarisation level.

Yet another aspect of the invention is a composition comprising hyperpolarised ¹³C-α-ketoisocaproic acid or hyperpolarised ¹³C-α-ketoisocaproate, a DNP agent according to formula (1) and optionally a paramagnetic metal ion, wherein said composition is obtained by dynamic nuclear polarisation of a composition as described in the previous paragraphs.

Yet another aspect of the invention is hyperpolarised ¹³C-α-ketoisocaproic acid or hyperpolarised ¹³C-α-ketoisocaproate, preferably TRIS-¹³C-α-ketoisocaproate or sodium ¹³C-α-ketoisocaproate. Preferred embodiments are hyperpolarised ¹³C1-α-ketoisocaproic acid or hyperpolarised ¹³C-α-ketoisocaproate, preferably TR1S-¹³C-α-ketoisocaproate or sodium ¹³C-α-ct-ketoisocaproate. The aforementioned compounds can be used as imaging agent (MR active agent) in the imaging medium according to the invention and said imaging medium can be used in the methods of ¹³C-MR detection according to the invention.

Yet another aspect of the invention is a method for producing hyperpolarised 1 -'C-(X-ketoisocaproic acid or hyperpolarised ¹³C-α-ketoisocaproate, the method comprising preparing a composition comprising ¹³C-α-ketoisocaproic acid or ¹³C-α-ketoisocaproate a DNP agent and optionally a paramagnetic metal ion and carrying out dynamic nuclear polarisation on said composition. The preparation of said composition and how to carry out dynamic nuclear on said composition is described in detail earlier in the application.

### Brief description of the drawings:

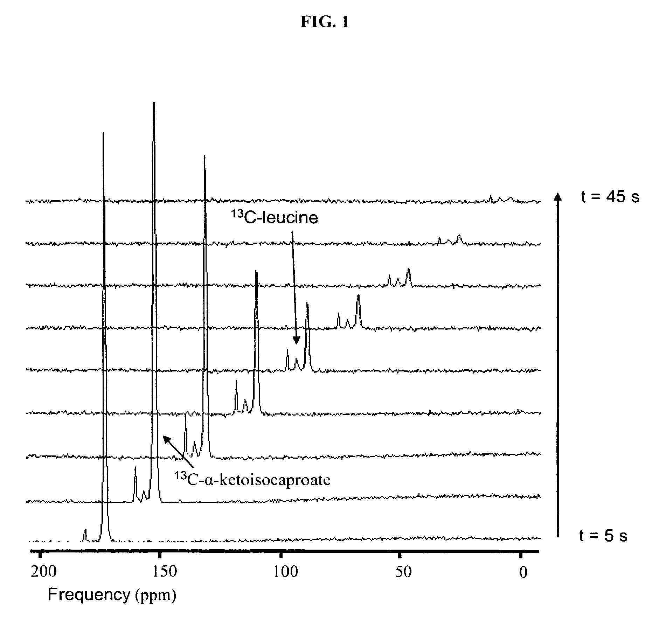

FIG. 1 shows the conversion of ¹³C-α-ketoisocaproate to ¹³C-α-leucine in mouse liver subsequent to administration of an imaging medium comprising hyperpolarised sodium ¹³C-α-ketoisocaproate. Time resolved ¹³C-NMR spectra were acquired and the signals of ¹³C-α-ketoisocaproate and ¹³C₁-leucine (176.8 ppm) were detected.

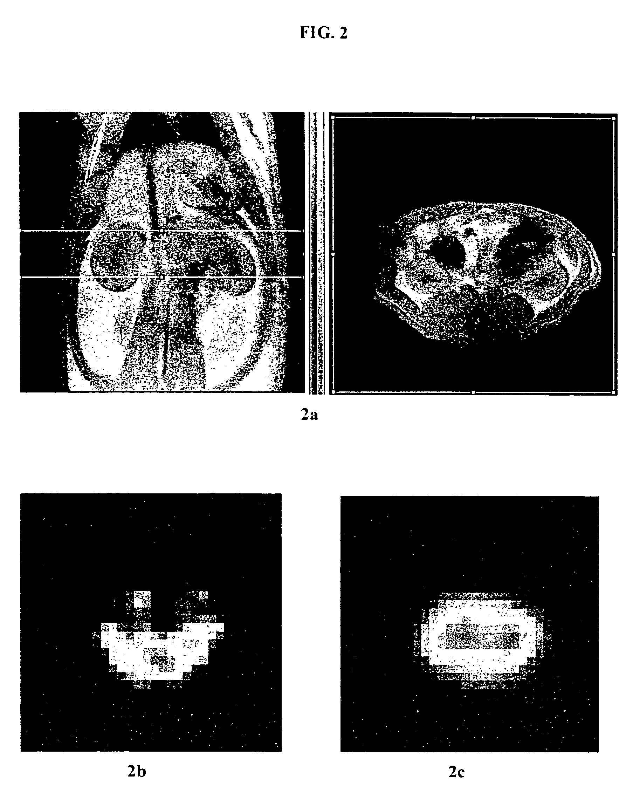

FIG. 2 depicts signal intensities of ¹³C-α-ketoisocaproate and ¹³C-leucine in a by-chemical shift image of primarily the kidneys in a healthy rat after administration of an imaging medium comprising hyperpolarised sodium ¹³C-α-ketoisocaproate. The slice selection is shown in FIG 2a. The ¹³C-αC-leucine signal distribution is shown in FIG. 2b and the ¹³C-α-ketoisocaproate signal distribution is shown in FIG. 2c.

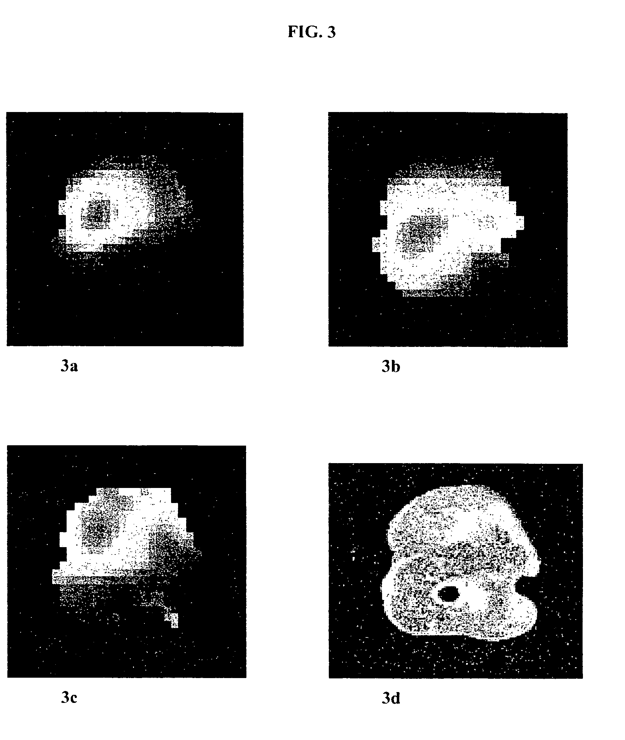

FIG. 3 depicts signal intensities of ¹³C-α-ketoisocaproate and ¹³C-leucine in a ¹³C-chemical shift image of a lymphoma mouse tumour (EL-4) subcutaneously grown on a mouse flank. The ¹³C-leucine signal distribution is shown in FIG. 3a and the ¹³C-α-ketoisocaproate signal distribution is shown in FIG. 3b. The ratio image (¹³C-α-leucine to ¹³C-α-ketoisocaproate) defines the tumour and is shown in FIG. 3c. A H reference was obtained with Omniscan^{™}-enhanced imaging, which is shown in FIG. 3d.

The invention is illustrated by the following examples.

### Examples

### Example 1 Production of an imaging medium comprising hyperpolarised TRIS-¹³C-α-ketoisocaproate

### Example 1a Preparation of TRIS-¹³C-α-ketoisocaproate

To a micro test tube were added sodium ¹³C-α-ketoisocaproate (Cambridge Isotopes, 151.2 mg, 0.987 mmol), TRIS (156.7 mg, 0.994 mmol) and 2 ml methanol. The test tube was sonicated and a white powder (NaCl) precipitated. The supernatant was taken up in a syringe and filtered through a syringe filter (0.45 µm) into another test tube containing 35 ml diethyl ether. The precipitation was centrifuged and the ether was removed by vacuum.

### Example 1b Dynamic nuclear polarisation of TRIS-¹³C₁-α-ketoisocaproate

The TRIS-¹³C₁-α-ketoisocaproate obtained in Example 1a (73.2 mg, 0.29 mmol) was dissolved in 50.0 mg of a mixture of tris(8-carboxy-2,2,6,6-(tetra(hydroxyethyl)-benzo-[1,2-4,5']-bis-(1,3)-dithiole-4-yl)-methyl sodium salt (trityl radical; 44.0 mg, 30.8 µmol) which had been synthesised according to Example 7 of WO-Al-98/39277 (44.0 mg, 30.8 µmol) and the Gd-chelate of 1,3,5-tris-(N-(DO3A-acetamido)-N-methyl-4-amino-2-methyl-phenyl)-[1,3,5]tria-zinane-2,4,6-trione (paramagnetic metal ion; 2.30 mg, 1.1 µmol) which had been synthesised according to Example 4 of WO-A-2007/064226 in glycerol (1543 µl, 1948 mg). The resulting composition was sonicated and gently heated to dissolve all compounds. The composition (80 µl 10 mM in trityl radical and 1 mM in Gd³⁺) was transferred with a pipette into a sample cup (probe-retaining container) which was quickly lowered into liquid nitrogen and then inserted into a DNP polariser. The composition was polarised under DNP conditions at 1.2 K in a 3.35 T magnetic field under irradiation with microwave (93.89 GHz). Polarisation was followed by solid state ¹³C-NMR and the solid state polarisation was determined to be 36%.

### Example 1c Dissolution and production of the imaging medium

After 120 minutes of dynamic nuclear polarisation, the obtained frozen polarised composition was dissolved in 6 ml phosphate buffer (pH 7.3, 40 mM). The pH of the final solution containing the dissolved composition was 7.3. The TRIS-¹³C-α-ketoisocaproate concentration in said final solution was 50 mM.

Liquid state polarisation was determined by liquid state ¹³C-NMR at 400 MHz to be 34 %.

### Example 2 Production of an imaging medium comprising hyperpolarised sodium ¹³C-α-ketoisocaproate

### Example 2a Dynamic nuclear polarisation of sodium ¹³C-α-ketoisocaproate

Sodium ¹³C-α-ketoisocaproate (19.5 mg, 0.126 mmol) was dissolved in 50.0 mg of a mixture of tris(8-carboxy-2,2,6,6-(tetra(hydroxyethyl)-benzo-[1,2-4,5']-bis-(1,3)-dithiole-4-yl)-methyl sodium salt (trityl radical; 44.0 mg, 30.8 µmol) which had been synthesised according to Example 7 of WO-Al-98/39277 (44.0 mg, 30.8 µmol) and the Gd-chelate of 1,3,5-tris-(N-(D03A-acetamido)-N-methyl-4-amino-2-methylphenyl)-[1,3,5]tria-zinane-2,4,6-trione (paramagnetic metal ion; 2.30 mg, 1.1 µmol) which had been synthesised according to Example 4 of WO-A-2007/064226 in glycerol (1543 µl, 1948 mg). To this solution 5 µl of water were added. The resulting composition was sonicated and gently heated to dissolve all compounds. The composition (110 µl, 12.5 mM in trityl radical and 1.3 mM in Gd³⁺) was transferred with a pipette into a sample cup which was quickly lowered into liquid nitrogen and then inserted into a DNP polariser. The composition was polarised under DNP conditions at 1.2 K in a 3.35 T magnetic field under irradiation with microwave (93.89 GHz). Polarisation was followed by solid state ¹³C-NMR and the solid state polarisation was determined to be approximately 30%.

### Example 2b Dissolution and production of the imaging medium

After 120 minutes of dynamic nuclear polarisation, the obtained frozen polarised composition was dissolved in 6 ml of phosphate buffer (pH 7.3, 40 mM) The pH of the final solution containing the dissolved composition was 7.3. The sodium ¹³C-α ketoisocaproate concentration in said final solution was 50 mM.

Liquid state polarisation was determined by liquid state ¹³C-NMR at 400 MHz to be 29 %.

### Example 3 Production of an imaging medium comprising hyperpolarised sodium ¹³C-α-ketoisocaproate

### Example 3a Preparation of ¹³C-α-ketoisocaproic acid

Sodium ¹³C-α-ketoisocaproate (210.0 mg, 1.37 mmol) was dissolved in a cooled solution of 500 µl concentrated H2SO4 in 2 ml water. The mixture was extracted 4 times with 6 ml diethyl ether. The organic phases were combined and dried over MgSO₄ The dried solution was filtered (0.45 µm syringe filter) to remove grains of MgSO₄ and the diethyl ether was removed under vacuum. 175 mg ¹³C₁-α-ketoisocaproic acid (98 %) were obtained.

### Example 3a Dynamic nuclear polarisation of ¹³C-α-ketoisocaproic acid

The trityl radical tris(8-carboxy-2,2,6,6-(tetra(methoxyethyl)-benzo-[1,2-4,5']-bis-(1,3)-dithiole-4-yl)-methyl sodium salt (1.18 mg, 0.74 mmol) which had been synthesised according to Example 1 of WO-A2-2006/011811 and 1.52 mg of an aqueous solution of the Gd-chelate of 1,3,5-tris-(N-(DO3A-acetamido)-N-methyl-4-amino-2-methyl-phenyl)-[1,3,5]tria-zinane-2,4,6-trione (paramagnetic metal ion, 14.5 µl/g solution) which had been synthesised according to Example 4 of WO-A-2007/064226 were dissolved in 49 µl ¹³C-α-ketoisocaproic acid (50.5 mg, 0.19 mmol). The resulting composition was sonicated and gently heated to dissolve all compounds. The composition (42 µl, 14 mM in trityl radical and 1.5 mM in Gd³⁺) was transferred with a pipette into a sample cup which was quickly lowered into liquid nitrogen and then inserted into a DNP polariser. The composition was polarised under DNP conditions at 1.2 K in a 3.35 T magnetic field under irradiation with microwave (93.89 GHz). Polarisation was followed by solid state ¹³C-NMR and the solid state polarisation was determined to be 27 %.

### Example 3c Dissolution and production of the imaging medium

After 90 minutes of dynamic nuclear polarisation, the obtained frozen polarised composition was dissolved in 6 ml of a solution prepared from 5.97 ml phosphate buffer (pH 7.3, 40 mM) and 30 µl aqueous NaOH solution (12 M). The pH of the final solution containing the dissolved composition was 7.3. The sodium ¹³C₁-α-ketoisocaproate concentration in said final solution was 50 mM.

Liquid state polarisation was determined by liquid state ¹³C-NMR at 400 MHz to be 25 %.

### Example 4 In vivo ¹³C-MR detection with a surface coil placed to detect the liver in a mouse using an imaging medium comprising hyperpolarised sodium ¹³C-α-ketoisocaproate

175 µl of an imaging medium comprising hyperpolarised sodium ¹³C₁-α-ketoisocaproate which was prepared as described in Example 1 was injected into a C57Bl/6 mouse over a time period of 6 s. The sodium ¹³C₁-α-ketoisocaproate concentration in said imaging medium was about 50 mM. A 12 mm surface coil (tuned for carbon) was positioned over the mouse to cover the liver region and signals of ¹³C-α-ketoisocaproate and ¹³C-leucine were detected by ¹³C-MR spectroscopy which was carried out in a 2.4 T Bruker spectrometer to generate a metabolic profile. A total of 10 ¹³C-spectra were acquired with a repetition time of 5 s and 30 degree RF pulses. The result is shown in Fig. 1. In this Example, the ¹³C-leucine signal is approximately 1% of the ¹³C-α-ketoisocaproate signal.

### Example 5 In vivo ¹³C-chemical shift imaging of a healthy rat kidney using an imaging medium comprising hyperpolarised sodium ¹³C₁-α-ketoisocaproate

2 ml of an imaging medium comprising hyperpolarised sodium ¹³C₁-α-ketoisocaproate which was prepared as described in Example 2 was injected into a Wistar rat over a time period of 6 s. The sodium ¹³C₁-α-ketoisocaproate concentration in said imaging medium was about 50 mM. A rat coil (tuned for carbon and proton) was positioned on the rat to cover the kidney region and the signals of ¹³C₁-α-ketoisocaproate and ¹³C-leucine were detected by ¹³C-MR spectroscopy which was carried out using a 2.4 T Bruker spectrometer to generate a metabolic profile of the kidney region. A ¹³C-chemical shift image was acquired with the following parameters: FOV 12.6x 12.6 mm² x 12 mm, matrix size 18x 18, 7 degree RF pulse, TR = 66 ms. Total acquisition time was 15 seconds and the chemical shift imaging started 18 seconds after the start of the injection of the imaging medium. A high resolution proton image was acquired for referencing. The results are shown in Fig 2.

### Example 6 In vivo ¹³C-chemical shift imaging with a surface coil placed over a subcutaneous mouse tymphoma (EL-4) using an imaging medium comprising hyperpolarised sodium ¹³C-α-ketoisocaproate

EL-4 cells were injected into a C57B1/6 mouse to generate a subcutaneous mouse lymphoma. 175 µl of an imaging medium comprising hyperpolarised sodium ¹³C-α-ketoisocaproate which was prepared as described in Example 2 was injected into the mouse over a time period of 6 s. The sodium ¹³C-α-ketoisocaproate concentration in said imaging medium was about 50 mM. A 20 mm surface coil (tuned for carbon) was positioned over the subcutaneous tumour and signals of ¹³C-α-ketoisocaproate and ¹³C-leucine were detected by ¹³C-MR spectroscopy which was carried out using a 2.4 T Bruker spectrometer to generate a metabolic profile of the tumour and the surrounding healthy tissue. A ¹³C-chemical shift image was acquired with the following parameters: FOV 35x35 mm² x10 mm, matrix size 16x16, 10 degree RF pulse, TR = 35 ms. Total acquisition time was 11 seconds (die to triggering on breathing) and the chemical shift imaging started 15 seconds after the start of the injection of the imaging medium. Omniscan^{™} (GE Healthcare) - enhanced proton imaging was performed to confirm the tumour position and perfusion. The results are shown in Fig. 3. The ¹³C-α-ketoisocaproate signal is the highest in the large blood vessels; however, the distribution of ¹³C-α-ketoisocaproate is seen over the whole field of view of the surface coil. The ¹³C-leucine distribution is confined to the tumour area and a ratio image (¹³C-leucine to ¹³C-α-ketoisocaproate) defines the tumour area which is confirmed and shown in the contrast-enhanced proton image.

## Claims

1. Imaging medium comprising Hyperpolarised ¹³C-α-ketoisocaproate.

2. The imaging medium according to claim 1 wherein said hyperpolarised ¹³C-α-ketoisocaproate is hyperpolarised ¹³C-α-ketoisocaproate.

3. The imaging medium according to claims I or 2 wherein said hyperpolarised ¹³C-α ketoisocaproate is hyperpolarised TRIS-¹³C-α-ketoisocaproate or hyperpolarised sodium ¹³C-α-ketoisocaproate.

4. The imaging medium according to claims 1 to 3 for use in *in vivo* or *in vitro* ¹³C-MR detection.

5. The imaging medium according to claim 4 wherein the use includes that signals of ¹³C-leucine and optionally of ¹³C-α-ketoisocaproate are detected.

6. The imaging medium according to claim 4 wherein the use includes that signals of ¹³CO₂ and/or ¹³C-bicarbonate and optionally of ¹³C-α-ketoisocaproate are detected.

7. The imaging medium according to claim 4 wherein the use includes that signals of ¹³C-α-leucine and ¹³CO₂ and/or ¹³C-α-bicarbonate and optionally of ¹³C-α-ketoisocaproate are detected.

8. The imaging medium according to claims 4 to 7 wherein said use is for *in vitro* ¹³C-MR detection and said signals are used to generate a metabolic profile of cells in a cell culture, of samples, of *ex vivo* tissue or of an isolated organ derived from a human or non-human animal being.

9. The imaging medium according to claim 4 wherein said use is for *in vivo* ¹³C-MR detection and said signals are used to generate a metabolic profile of a living human or non-human animal being.

10. The imaging medium according to claims 8 and 9 wherein the metabolic profile is used for identifying diseases, preferably cancer.

11. Composition comprising ¹³C-α-ketoisocaproate or ¹³C-α-ketolsocaproic acid, a DNP agent and optionally a paramagnetic metal ion, wherein the DNP agent is a trityl radical of formula (1) 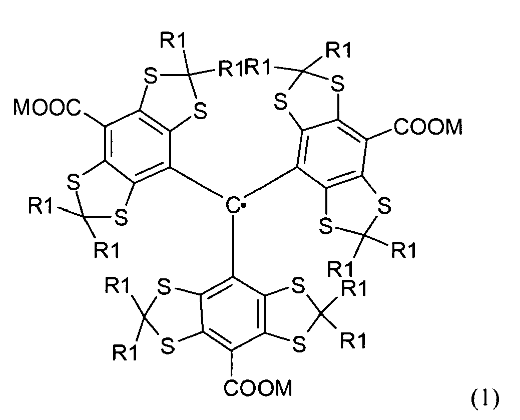 wherein
M represents hydrogen or one equivalent of a cation; and
R1 which is the same or different represents a straight chain or branched C₁-C₆-alkyl group optionally substituted by one or more hydroxyl groups or a group-(CH₂)ₙ-X-R2,
wherein n is 1, 2 or 3;
X is O or S; and
R2 is a straight chain or branched C₁-C₄-alkyl group, optionally substituted by one or more hydroxyl groups.

12. The composition according to claim 11 wherein said paramagnetic metal ion is present and is a paramagnetic chelate comprising Gd³⁺.

13. The composition according to claims 11 to 12 for use in dynamic nuclear polarisation.

14. Composition comprising hyperpolarised ¹³C-α-ketoisocaproate or ¹³C-α-ketoisocaproic acid, a DNP agent and optionally a paramagnetic metal ion, wherein said composition is obtained by dynamic nuclear polarisation of the composition of claims 11 to 12.

## Patentansprüche

1. Abbildungsmedium, umfassend hyperpolarisiertes ¹³C-α-Ketoisocaproat.

2. Abbildungsmedium nach Anspruch 1, wobei das hyperpolarisierte ¹³C-α-Ketoisocaproat hyperpolarisiertes ¹³C-α-Ketoisocaproat ist.

3. Abbildungsmedium nach Anspruch 1 oder 2, wobei das hyperpolarisierte ¹³C-α-Ketoisocaproat hyperpolarisiertes TRIS-¹³C-α-Ketoisocaproat oder hyperpolarisiertes Natrium-¹³C-α-Ketoisocaproat ist.

4. Abbildungsmedium nach Ansprüchen 1 bis 3 zur Verwendung bei *in vivo* oder *in vitro* ¹³C-MR-Erfassung.

5. Abbildungsmedium nach Anspruch 4, wobei die Verwendung einschließt, dass Signale von ¹³C-Leucin und gegebenenfalls von ¹³C-α-Ketoisocaproat erfasst werden.

6. Abbildungsmedium nach Anspruch 4, wobei die Verwendung einschließt, dass Signale von ¹³CO₂ und / oder ¹³C-Bicarbonat und gegebenenfalls von ¹³C-α-Ketoisocaproat erfasst werden.

7. Abbildungsmedium nach Anspruch 4, wobei die Verwendung einschließt, dass Signale von ¹³C-Leucin und ¹³CO₂ und / oder ¹³C-Bicarbonat und gegebenenfalls von ¹³C-α-Ketoisocaproat erfasst werden.

8. Abbildungsmedium nach Ansprüchen 4 bis 7, wobei der Verwendungszweck *in vitro* ¹³C-MR-Erfassung ist und die Signale genutzt werden, um ein metabolisches Profil zu erzeugen von Zellen in einer Zellkultur, von Proben, von ex *vivo* Gewebe oder von einem isolierten Organ, stammend von einem menschlichen oder nicht menschlichen Lebewesen.

9. Abbildungsmedium nach Anspruch 4, wobei der Verwendungszweck *in vivo* ¹³C-MR-Erfassung ist und die Signale genutzt werden, um ein metabolisches Profil von einem lebenden menschlichen oder nicht menschlichen Lebewesen zu erzeugen.

10. Abbildungsmedium nach Ansprüchen 8 und 9, wobei das metabolische Profil zur Erkennung von Krankheiten, vorzugsweise von Krebs, verwendet wird.

11. Zusammensetzung, umfassend ¹³C-α-Ketoisocaproat oder ¹³C-α-Ketoisocapronsäure, ein DNP-Agens und gegebenenfalls ein paramagnetisches Metallion, wobei das DNP-Agens ein Tritylradikal der Formel (1) ist, 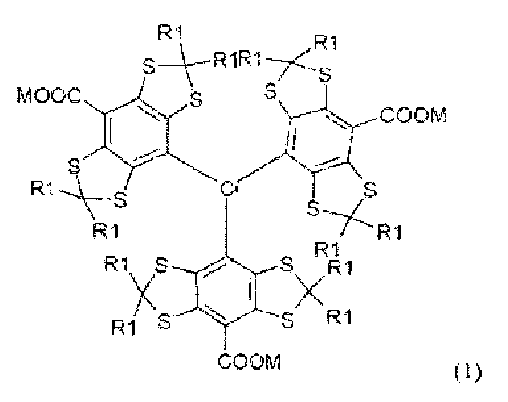 worin
M Wasserstoff oder ein Äquivalent eines Kations darstellt; und
R1, das gleich oder verschieden ist, eine geradkettige oder verzweigte C₁-C₆-Alkylgruppe darstellt, die gegebenenfalls durch eine oder mehrere Hydroxygruppen oder eine Gruppe -(CH₂)ₙ-X-R2 substituiert ist,
worin n für 1, 2 oder 3 steht;
X für O oder S steht; und
R2 für eine geradkettige oder verzweigte C₁-C₄-Alkylgruppe steht, die gegebenenfalls durch eine oder mehrere Hydroxygruppen substituiert ist.

12. Zusammensetzung nach Anspruch 11, wobei das paramagnetische Metallion vorhanden ist und ein paramagnetisches Chelat ist, das Gd³⁺ umfasst.

13. Zusammensetzung nach Ansprüchen 11 bis 12 zur Verwendung in dynamischer Kernpolarisation.

14. Zusammensetzung, umfassend hyperpolarisierte(s) ¹³C-α-Ketoisocaproat oder ¹³C-α-Ketoisocapronsäure, ein DNP-Agens und gegebenenfalls ein paramagnetisches Metallion, wobei die Zusammensetzung durch dynamische Kernpolarisation der Zusammensetzung der Ansprüche 11 bis 12 erhalten wird.

## Revendications

1. Milieu d'imagerie comprenant du ¹³C-α-cétoisocaproate hyperpolarisé.

2. Milieu d'imagerie selon la revendication 1 dans lequel ledit ¹³C-α-cétoisocaproate hyperpolarisé est du ¹³C-α-cétoisocaproate hyperpolarisé.

3. Milieu d'imagerie selon les revendications 1 ou 2 dans lequel ledit ¹³C-α-cétoisocaproate hyperpolarisé est du TRIS-¹³C-α-cétoisocaproate hyperpolarisé ou du sodium ¹³C-α-cétoisocaproate hyperpolarisé.

4. Milieu d'imagerie selon les revendications 1 à 3 destiné à une utilisation en détection par RM de ¹³C in vivo ou in vitro.

5. Milieu d'imagerie selon la revendication 4 dans lequel l'utilisation comporte la détection des signaux de ¹³C-leucine et, en variante, de ¹³C-α-cétoisocaproate.

6. Milieu d'imagerie selon la revendication 4 dans lequel l'utilisation comporte la détection des signaux de ¹³CO₂ et/ou de ¹³C-bicarbonate et, en variante, de ¹³C-α-cétoisocaproate.

7. Milieu d'imagerie selon la revendication 4 dans lequel l'utilisation comporte la détection des signaux de ¹³C-leucine et de ¹³CO₂ et/ou de ¹³C-bicarbonate et, en variante, de ¹³C-α-cétoisocaproate.

8. Milieu d'imagerie selon les revendications 4 à 7 dans lequel ladite utilisation est destinée à assurer la détection par RM de ¹³C in vitro et lesdits signaux sont utilisés afin de produire un profil métabolique des cellules dans une culture cellulaire d'échantillons, de tissu ex vivo ou d'un organe isolé dérivé d'un être animal humain ou non humain.

9. Milieu d'imagerie selon la revendication 4 dans lequel ladite utilisation est destinée à assurer la détection par RM de ¹³C in vivo et lesdits signaux sont utilisés afin de produire d'un profil métabolique d'un être animal humain ou non humain vivant.

10. Milieu d'imagerie selon les revendications 8 et 9 dans lequel le profil métabolique est utilisé afin d'identifier des maladies, de préférence, un cancer.

11. Composition comprenant du ¹³C-α-cétoisocaproate ou de l'acide ¹³C-α-cétoisocaproïque, un agent de DNP (polarisation nucléaire dynamique) et, en variante, un ion métallique paramagnétique, l'agent de DNP est un radical trityle selon la formule (1 ): 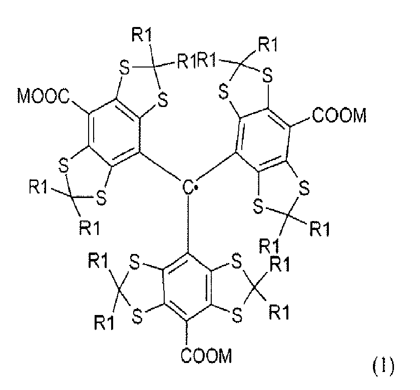 dans laquelle
M représente de l'hydrogène ou un équivalent d'un cation ; et
R1 qui est identique ou différent, représente un groupe alkyle en C₁ à C₆ à chaîne linéaire ou ramifiée, en variante, substitué par un ou plusieurs groupes hydroxyles ou un groupe -(CH₂)ₙ-X-R2,
dans lequel n est égal à 1, 2 ou 3 ;
X représente O ou S ; et
R2 représente un groupe alkyle en C₁ à C₄ linéaire ou ramifié, en variante, substitué par un ou plusieurs groupes hydroxyles.

12. Composition selon la revendication 11 dans laquelle ledit ion métallique paramagnétique est présent et est un chélate paramagnétique comprenant du Gd³⁺.

13. Composition selon les revendications 11 ou 12 destinée à une utilisation en polarisation nucléaire dynamique.

14. Composition comprenant du ¹³C-α-cétoisocaproate hyperpolarisé ou de l'acide ¹³C-α-cétoisocaproïque, un agent de DNP et, en variante, un ion métallique paramagnétique, dans laquelle ladite composition est obtenue par la polarisation nucléaire dynamique de la composition selon les revendications 11 à 12.
